# EUROPEAN PATENT APPLICATION

(11) **EP 2 823 824 A1**
(43) Date of publication of application: **14.01.2015**
(21) Application number: 13757952.0
(22) Date of filing: 08.03.2013
(51) Int. Cl.: A61K 39/00, A61K 9/08, A61K 9/19, A61K 39/39, A61K 47/02, A61K 47/18, A61P 35/00, A61P 37/04

(54) **PHARMACEUTICAL COMPOSITION CONTAINING PEPTIDE**

(30) Priority: 09.03.2012 JP 2012072352
(71) Applicant: OncoTherapy Science, Inc., Kawasaki-shi Kanagawa 213-0012 (JP)
(72) Inventor: HIMI, Takeo, Toyama-shi Toyama 939-2721 (JP); WATANABE, Takeshi, Toyama-shi Toyama 939-2721 (JP); NOGAMI, Toshihiro, Toyama-shi Toyama 939-2721 (JP); SAKAI, Masami, Kawasaki-shi Kanagawa 213-0012 (JP); WATANABE, Yukino, Kawasaki-shi Kanagawa 213-0012 (JP)
(74) Representative: Woods, Geoffrey Corlett
(86) International application number: PCT/JP2013/056429
(87) International publication number: WO 2013/133405

(57) **Abstract**

This invention provides a method for preparing a formulation comprising one or more types of peptides with the use of the same solvent in a simple manner without the need for selecting an adequate solvent for each peptide in accordance with its solubility. The invention provides a pharmaceutical composition comprising one or more types of peptides as active ingredients, a basic amino acid, and/or a base, and a method for producing such composition.

## Description

### Technical Field

The present invention relates to a pharmaceutical composition containing peptides and a method for producing the same.

### Background Art

Injection formulations containing peptides cannot be subjected to the process of final sterilization involving the use of high pressure steam since peptides are heat-unstable. A process of aseptic filtration is required in order to achieve sterilization. Aseptic filtration is generally performed through a membrane filter with a certified absolute pore size of 0.22 µm or less. Accordingly, it is necessary to prepare a peptide solution in which peptides are completely dissolved before filtration. However, the solubility of different peptides differ based on amino acid sequence, and such difference necessitates selection of an adequate solvent in accordance with the solubility of the peptide of interest. It is particularly difficult to completely dissolve a peptide with high hydrophobicity in a polar solvent, and selection of a solvent is very laborious. While solubility can be enhanced by changing the pH level, the resulting pH level often deviates from a level suitable for an injection formulation, or the resulting peptide often becomes unstable.

In recent years, also, a peptide vaccine preparation containing, as active ingredients, a plurality of types of peptides has drawn attention, in addition to a peptide vaccine preparation containing only one type of peptide. Such peptide vaccine preparation is considered to be particularly useful for cancer therapy.

A peptide vaccine preparation for cancer therapy comprises, as an active ingredient, a T cell epitope peptide of a tumor-specific antigen, in order to induce immune responses specific to a cancer cell (e.g., Patent Document 1). A tumor-specific antigen from which such T cell epitope peptide is derived has been identified through extensive expression analysis using clinical samples of cancer patients as an antigen that is expressed at a high level specific to a cancer cell but is not substantially expressed in normal cells for each cancer type (e.g., Patent Document 2). However, such tumor-specific antigen that has been identified in the manner described above is not always expressed at a high level in all patients and in all cancer cells because of the diversity of cancer cells. That is, an antigen that is expressed at a high level in cancer of a particular patient may not be expressed to such an extent in cancer of another patient. In addition, it is known that cancer cells constitute a population of heterogeneous cells at the cellular level in a single patient (Non-Patent Document 1), and an antigen that is expressed in a particular cancer cell may not be expressed in another cancer cell. Accordingly, a vaccine preparation containing only one type of T cell epitope peptide may not be able to exert satisfactory antitumor effects on every patient. In addition, some cancer cells may not be killed in a patient who has achieved antitumor effects. If a vaccine preparation contains a plurality of types of T cell epitope peptides, in contrast, it is highly likely that a cancer cell expresses an antigen corresponding to any such plurality of peptides. Accordingly, antitumor effects can be expected for more patients, and the risk of cancer cells remaining undamaged can be reduced.

The effects of a vaccine preparation containing a plurality of types of T cell epitope peptides as described are enhanced as the number of types of T cell epitope peptides to be incorporated is increased. In order to incorporate effective amounts of a plurality of types of T cell peptides, however, peptide content per unit amount is also increased. Thus, it becomes more difficult to completely dissolve all peptides. Because of the presence of a plurality of types of peptides with different properties, in addition, it becomes more difficult to maintain all peptides in stable conditions.

For example, European Patent Publication (EP 2111867) (Patent Document 3) discloses a lyophilized vaccine formulation for cancer therapy that contains a plurality of types of T cell epitope peptides. In the process of preparation of peptide solutions for such lyophilized formulation before the process of lyophilization, each peptide is dissolved in an adequate solvent in accordance with its solubility. Then, in order to prevent peptides from precipitating, the peptide solutions are mixed in a designated order. As the number of types of peptides to be incorporated increases, accordingly, it becomes more laborious to select an adequate solvent for each peptide and determine the order for mixing peptide solutions.

In order to avoid such difficulty when producing formulations, a plurality of types of vaccine preparations each containing one type of T cell epitope peptide may be administered to a patient. When a plurality of types of vaccine preparations are to be administered, however, vaccines need to be inoculated at more than one site in a body, and such inoculations put more stress on a patient. Also, peptide vaccine preparations often cause cutaneous reactions referred to as delayed-type hypersensitivity (DTH) reactions after inoculation. Cutaneous reactions occurring at more than one site in a body increase the sense of discomfort in a patient. In order to reduce the stress on a patient by vaccine inoculation, accordingly, a vaccine preparation preferably contains a plurality of types of T cell epitope peptides. In the case of administration of a plurality of types of vaccine preparations each containing one type of epitope peptide, also, it is necessary to select an adequate solvent for each peptide when preparing peptide preparations.

### Prior Art Documents

Patent Documents:
Patent Document 1: WO 2008/102557
Patent Document 2: WO 2004/031413
Patent Document 3: EP 2 111 867

Non-Patent Documents:
Non-Patent Document 1: Kai Wang et al., BMC Bioinformatics 2009, 10: 12

### Summary of the Invention

### Problem to be Solved by the Invention

It is an object of the present invention to provide a means for preparing a formulation comprising one or more types of peptides with the use of the same solvent in a simple manner without the need for selecting an adequate solvent for each peptide in accordance with its solubility.

### Means for Solving the Problem

The present inventors have conducted concentrated studies in order to attain the above object. As a result, they discovered that an aqueous solution containing sodium carbonate and/or arginine would be capable of dissolving various types of peptides with different degrees of solubility in water. In addition, they discovered that such aqueous solution would be suitable for preparing an injection formulation containing a plurality of types of peptides. Further, they confirmed that the pH level of an injection formulation prepared with the use of such aqueous solution was within an adequate range for an injection formulation. The present invention has been completed based on such findings.

### Specifically, the present invention includes the following.

[1] A pharmaceutical composition comprising one or more types of peptides as active ingredients and a basic amino acid and/or a base.
[2] The pharmaceutical composition according to [1], wherein the basic amino acid is one basic amino acid or a combination of two or more basic amino acids selected from the group consisting of arginine, lysine, ornithine, histidine, hydroxylysine, and a salt of any thereof.
[3] The pharmaceutical composition according to [1] or [2], wherein the base is one base or a combination of two or more bases selected from the group consisting of sodium carbonate, potassium carbonate, sodium bicarbonate, potassium bicarbonate, sodium hydroxide, potassium hydroxide, triethanolamine, trometamol, and meglumine.
[4] The pharmaceutical composition according to any of [1] to [3], wherein the peptide is a T cell epitope peptide.
[5] The pharmaceutical composition according to any of [1] to [3], wherein the peptide is a peptide composed of two or more types of T cell epitope peptides linked to each other directly or via a linker.
[6] The pharmaceutical composition according to [4] or [5], wherein the T cell epitope peptide is one or more types of peptides each consisting of the amino acid sequence as shown in SEQ ID NOs: 1, 2, 3, 4, or 5.
[7] The pharmaceutical composition according to [4] or [5], wherein the T cell epitope peptide is all types of peptides each consisting of the amino acid sequence as shown in SEQ ID NOs: 1, 2, 3, or 4.
[8] The pharmaceutical composition according to any of [4] to [7], which is used for inducing an immune response to a tumor.
[9] The pharmaceutical composition according to [8], which is used for prevention or treatment of cancer.
[10] The pharmaceutical composition according to any of [1] to [9], which is prepared in the form of a parenteral formulation.
[11] The pharmaceutical composition according to [10], wherein the parenteral formulation is an injection preparation.
[12] A lyophilized formulation of the pharmaceutical composition according to any of [1] to [11].
[13] A method for producing a pharmaceutical composition, which is the injection preparation according to [11], comprising a step of dissolving one or more types of peptides in an aqueous solution comprising a basic amino acid and/or a base.
[14] A method for producing the lyophilized formulation according to [12], which comprises the following steps of:
   (1) dissolving one or more types of peptides in an aqueous solution comprising a basic amino acid and/or a base; and
   (2) lyophilizing the peptide solution prepared in (1).
[15] The method for producing the lyophilized formulation according to [14], wherein the peptide is all types of peptides each consisting of the amino acid sequence as shown in SEQ ID NOs: 1, 2, 3, or 4.
[16] A kit comprising (a) and (b) below:
   (a) the lyophilized formulation according to [12]; and
   (b) a reconstituting solution for the lyophilized formulation of (a).
[17] A kit comprising (a) and (b) below:
   (a) a lyophilized formulation of the pharmaceutical composition according to [6] or [7]; and
   (b) a reconstituting solution for the lyophilized formulation of (a), comprising a peptide consisting of the amino acid sequence as shown in SEQ ID NO: 5.
[18] The kit according to [16] or [17], which further comprises (c) below:
   (c) one or more types of adjuvants.
[19] A kit comprising (a) and (b) below:
   (a) the pharmaceutical composition according to any of [1] to [11]; and
   (b) one or more types of adjuvants.

This patent application claims priority from Japanese Patent Application No. 2012-072352 filed on March 9, 2012, and includes some or all of the content as disclosed in the description thereof.

### Effects of the Invention

The present invention provides a pharmaceutical composition comprising one or more types of peptides that can be prepared with the use of the same solvent in a simple manner without the need for selecting an adequate solvent for each peptide in accordance with its solubility. Since the pharmaceutical composition of the present invention can comprise a larger number of types of peptides incorporated therein, such composition can be provided in the form of, for example, a vaccine formulation comprising a plurality of T cell epitope peptides. Thus, satisfactory antitumor effects can be achieved, and use of such vaccine preparation for cancer therapy is very effective.

### Brief Description of the Drawings

Fig. 1 shows stability of a peptide-containing lyophilized formulation according to an embodiment of the present invention.
Fig. 2 shows the stability of a peptide-containing lyophilized formulation according to an embodiment of the present invention 24 months later predicted via regression analysis.

### Embodiments for Carrying out the Invention

Hereafter, the present invention is described in detail.

The article "a," "an," or "the" used herein refers to "at least one," unless otherwise specified.

The term "peptide" used herein refers to a polymer of amino acid residues and more specifically, to an amino acid polymer composed of natural amino acid residues, an amino acid polymer composed of natural amino acid residues and one or more non-natural amino acid residues such as amino acid analogs or mimics, or an amino acid polymer composed of non-natural amino acid residues.

The term "amino acid" used herein refers to a natural amino acid, a synthetic amino acid, or an amino acid analog or mimic that functions in the same manner as a natural amino acid. An amino acid may be either an L-amino acid or a D-amino acid. The term "natural amino acid" refers to an amino acid encoded by a genetic code and an amino acid subjected to post-translational modification in the cell (e.g., hydroxyproline, γ-carboxyglutamic acid, or O-phosphoserine). The term "amino acid analog" refers to a compound having the same basic chemical structure as a natural amino acid (i.e., an alpha carbon bound to a hydrogen, a carboxy group, an amino acid group, and an R group) but with a modified R group or backbone (e.g., homoserine, norleucine, methionine, sulfoxide, or methionine methyl sulfonium). The term "amino acid mimic" refers to a compound that has a structure different from that of a general amino acid but has functions equivalent thereto. Herein, amino acids are represented by generally known three-letter notations or one-letter notations recommended by the IUPAC-IUB Commission on Biochemical Nomenclature.

The term "T cell epitope peptide" used herein refers to a peptide that is bound to a major histocompatibility complex (MHC) class I or class II molecule, presented on a cell surface, and recognized by a T cell via a T cell receptor. A T cell epitope peptide that binds to an MHC class I molecule generally consists of 8 to 14 amino acids and typically consists of 9 or 10 amino acids. A peptide presented on a cell surface by the MHC class I molecule is recognized by a CD8-positive T cell, and it activates the CD8-positive T cell to induce a cytotoxic T lymphocyte (CTL). A T cell epitope peptide that binds to an MHC class II molecule generally consists of 12 to 30 amino acids and typically consists of 15 to 24 amino acids. A peptide presented on a cell surface by the MHC class II molecule is recognized by a CD4-positive T cell, and it activates the CD4-positive T cell.

The term "tumor-specific antigen" used herein refers to an antigen that is expressed in a tumor cell but is not expressed or hardly expressed in a normal cell. A tumor-specific antigen may be expressed in a particular tumor cell (e.g., a gastric cancer cell) or in any of a variety of tumor cells. Also, a cancer-testis antigen that is expressed specifically in a tumor cell and in the testis is within the scope of the "tumor-specific antigen."

### 1. The pharmaceutical composition of the present invention

The pharmaceutical composition of the present invention comprises one or more types of peptides as active ingredients, and a basic amino acid and/or a base.

Any biologically active peptides may be incorporated into the pharmaceutical composition of the present invention without particular limitation. Examples of peptides that can be incorporated into the pharmaceutical composition of the present invention include, but are not limited to, immunogenic peptides such as T cell epitopes and antibody epitopes, dominant negative peptides, aptamers, enzymes, antibodies, antibody fragments such as scFv, Fab, F(ab')₂, and Fv, hormones, nerve transmitter substances, opioid peptides, autacoids, and cytokines.

According to a preferable embodiment, the pharmaceutical composition of the present invention comprises, as active ingredients, one or more types of T cell epitope peptides. A plurality of MHC class I-binding T cell epitope peptides and MHC class II-binding T cell epitope peptides have been identified in the past. Thus, those known T cell epitope peptides can be used in the pharmaceutical composition of the present invention alone or in appropriate combination of two or more types thereof. When two or more types of T cell epitope peptides are used in combination, such T cell epitope peptides may be linked to each other directly or via a linker. Examples of linkers that can be used include, but are not limited to, AAY (P. M. Daftarian et al., J. Trans. Med., 2007, 5: 26), AAA and NKRK (R. P. M. Sutmuller et al., J. Immunol., 2000, 165: 7308-7315), and polylysine (S. Ota et al., Can. Res., 62, 1471-1476; K. S. Kawamura et al., J. Immunol., 2002, 168: 5709-5715).

Examples of T cell epitope peptides that can be used for the pharmaceutical composition of the present invention include, but are not limited to, peptides derived from tumor-specific antigens, peptides derived from HIV or other virus antigens, and peptides derived from allergens, with T cell epitope peptides derived from tumor-specific antigens being preferable. A plurality of types of T cell epitope peptides derived from tumor-specific antigens that bind to human MHC molecules; i.e., human leukocyte antigens (HLAs), have been identified. Human MHC class I molecules are HLA-A, HLA-B, and HLA-C, and human MHC class II molecules are HLA-DR, HLA-DQ, and HLA-DP. T cell epitope peptides contained in the pharmaceutical composition of the present invention may be T cell epitope peptides that bind to any of such HLA antigens, and T cell epitope peptides that bind to HLA-A antigens are preferable. Up to the present, many molecules that bind to HLA-A antigens have been identified. Examples of T cell epitope peptides derived from tumor-specific antigens that bind to HLA-A2 or HLA-A24, which are HLA-A antigen alleles, include peptides described in WO 2003/104275, WO 2004/018667, WO 2004/024766, WO 2006/090810, WO 2006/093030, WO 2007/013576, WO 2007/018199, WO 2008/047473, WO 2008/102557, WO 2009/025117, WO 2009/025196, WO 2009/150822, WO 2009/153992, WO 2010/013485, WO 2010/047062, WO 2010/064430, WO 2010/070877, WO 2010/073551, WO 2010/095428, WO 2010/100878, WO 2010/106770, WO 2011/067920, and WO 2011/089921. It should be noted that T cell epitope peptides that can be used for the pharmaceutical composition of the present invention are not limited thereto.

A peptide used for the pharmaceutical composition of the present invention may be a peptide consisting of an amino acid sequence derived from a natural protein or a modified peptide thereof. In general, modification of 1, 2, or several amino acid residues in a particular peptide does not influence functions of the peptide, and such modifications occasionally enhance desirable functions of an original peptide. In fact, a modified peptide, which is a peptide consisting of an amino acid sequence having modification (i.e., substitution, deletion, addition, and/or insertion) of 1, 2, or several amino acid residues 1, 2, or several amino acid residues relative to the original sequence, is known to retain biological activity of the original peptide (Mark et al., Proc. Natl. Acad. Sci., U.S.A., 1984, 81: 5662-6; Zoller and Smith, Nucleic Acids Res., 1982, 10: 6487-500; and Dalbadie-McFarland et al., Proc. Natl. Acad. Sci., U.S.A., 1982, 79: 6409-13). As with the peptide identified as the T cell epitope peptide derived from a natural tumor-specific antigen, accordingly, such modified peptide can be preferably used for the pharmaceutical composition of the present invention. The term "several" used herein refers to preferably 10 or fewer, and more preferably 5, 4, or 3.

A person skilled in the art would understand substitutions of amino acid residues that results in conservation of properties of the original peptide. Such substitutions of amino acid residues are referred to as "conservative substitutions". Conservative substitutions by functionally similar amino acids are a technique well known in the art. The term "conservative amino acid substitution" refers to substitution between amino acids having similar properties, such as constitutional properties, electrical properties, or properties related to polarity or hydrophobicity. Such properties can be classified in terms of, for example, similarity of amino acid side chains. Amino acids having basic side chains are lysine, arginine, and histidine, amino acids having acidic side chains are aspartic acid and glutamic acid, amino acids having non-charged polar side chains include glycine, asparagine, glutamine, serine, threonine, tyrosine, and cysteine, amino acids having hydrophobic side chains include alanine, valine, leucine, isoleucine, proline, phenylalanine, and methionine, amino acids having branched side chains are threonine, valine, leucine, and isoleucine, and amino acids having aromatic side chains are tyrosine, tryptophan, phenylalanine, and histidine. The 8 groups below each comprise amino acids that are recognized as capable of conservative substitution in the art (see, for example, Creighton, Proteins, 1984):
1) alanine (A) and glycine (G);
2) aspartic acid (D) and glutamic acid (E);
3) asparagine (N) and glutamine (Q);
4) arginine (R) and lysine (K);
5) isoleucine (I), leucine (L), methionine (M), and valine (V);
6) phenylalanine (F), tyrosine (Y), and tryptophan (W);
7) serine (S) and threonine (T); and
8) cysteine (C) and methionine (M).

Thus, the pharmaceutical composition of the present invention can comprise a peptide obtained by modification of the original peptide through conservative substitution as an active ingredient. However, the modified peptides that can be used for the pharmaceutical composition of the present invention are not limited thereto, and non-conservative modification may be employed, provided that the modified peptide retains properties of the original peptide. The term "property" used herein refers to biological activity of a peptide that is expected in a pharmaceutical composition. If a peptide is a T cell epitope peptide, for example, the type of modification and the number of modified amino acid residues are not limited, provided that the modified peptide retains the CTL-inducting activity of the original peptide.

When peptides are modified via substitution, addition, and/or insertion of amino acid residues, modifications may be carried out with L-amino acids, D-amino acids, or non-natural amino acids such as amino acid analogs or mimics. For example, a technique of introduction of D-amino acids or non-natural amino acids such as amino acid analogs or mimics for the purpose of enhancement of the *in vivo* stability of peptides is known in the art. As long as the modified peptides retain properties of the original peptides, accordingly, peptides may be modified peptides that contain D-amino acids and/or non-natural amino acids such as amino acid analogs or mimics.

In addition to modification described above, other substances may be linked to peptides, provided that properties of the original peptides are retained. Examples of other substances that can be linked to peptides include another peptide, fat, sugar, a sugar chain, an acetyl group, and a natural or synthetic polymer. As long as properties of the original peptide are retained, a peptide may be subjected to modification, such as sugar chain addition, side chain oxidation, and phosphorylation.

The pharmaceutical composition of the present invention can comprise a peptide that is hardly soluble in a polar solvent such as water as an active ingredient. For example, it is difficult to dissolve an effective amount of a peptide with solubility in water at 25 degrees C and at an atmospheric pressure of less than 1 mg/ml (e.g., less than 0.9 mg/ml, less than 0.8 mg/ml, less than 0.7 mg/ml, less than 0.6 mg/ml, or less than 0.5 mg/ml) in a solvent such as sterile water prepared for injection (e.g., Water for Injection defined by the Japanese Pharmacopoeia). However, the pharmaceutical composition of the present invention can comprise an effective amount of a peptide with low solubility in water in the form of an aqueous solution. In the pharmaceutical composition of the present invention, accordingly, the solubility of a peptide in water is not particularly limited.

The pharmaceutical composition of the present invention can comprise a plurality of types of peptides with different degrees of solubility in water. In the case of a pharmaceutical composition for inducing immune responses, for example, a pharmaceutical composition containing a plurality of types of peptides can occasionally exert more preferable effects than a pharmaceutical composition containing a single type of peptide. When an immune response to a tumor is to be induced, for example, use of a plurality of types of antigen peptides is often preferable because of the diversity of tumor cells. Since a tumor is composed of a population of heterogeneous cells, an antigen that is expressed in a particular tumor cell may not be expressed in another tumor cell. If a plurality of types of antigen peptides are used, it is highly likely that a tumor cell of interest would express an antigen corresponding to any of such plurality of peptides. Accordingly, enhanced effects of inducing immune responses to tumors can be expected. When an injection solvent is used, incorporation of a plurality of types of antigen peptides leads to an increase in the total amount of peptides. Thus, it is often difficult to dissolve all peptides. However, the pharmaceutical composition of the present invention can comprise effective amounts of a plurality of types of peptides with different degrees of solubility in water in the state of an aqueous solution. For example, a pharmaceutical composition containing a plurality of types of T cell epitope peptides derived from tumor-specific antigens is a preferable embodiment of the pharmaceutical composition according to the present invention aimed at induction of an immune response to a tumor.

According to a preferable embodiment, a pharmaceutical composition aimed at induction of an immune response to a particular tumor comprises a plurality of types of T cell epitope peptides derived from the tumor-specific antigens that are known to be expressed at a high level in the tumor of interest. For example, a pharmaceutical composition that induces an immune response to gastric cancer can contain a plurality of types of T cell epitope peptides derived from the tumor-specific antigens that are known to be expressed at a high level in gastric cancer. The same applies to other types of cancers. The term "expressed at a high level" used herein refers to a higher expression level in the tumor cells than in cells of a corresponding normal organ. When the expression level of a particular gene in a tumor cell is 1.5 times or more higher than that in the cells of the normal organ, for example, such gene is considered to be expressed at a high level in the tumor cell. The expression level in a tumor cell is preferably at least 2 times and more preferably at least 3 times higher than that in a normal cell.

Examples of tumor-specific antigens known to be expressed at a high level in gastric cancer cells include, but are not limited to, CDH3, URLC10, CXDRL1, GCUD1, VEGR1, VEGFR2, MPHOSPH1, DEPDC1, KIF20A, and FOXM1. The pharmaceutical composition of the present invention comprising a plurality of types of T cell epitope peptides selected from among the T cell epitope peptides derived from the tumor-specific antigens above is suitable for induction of an immune response to gastric cancer. When the pharmaceutical composition of the present invention is aimed at induction of an immune response to gastric cancer, for example, T cell epitope peptides derived from tumor-specific antigens contained in the pharmaceutical composition of the present invention are preferably one or more peptides selected from among peptides each consisting of the amino acid sequence as shown in SEQ ID NO: 1, 2, 3, 4, or 5. Peptides each consisting of the amino acid sequence as shown in SEQ ID NO: 1, 2, 3, 4, or 5 may be variant peptides thereof, provided that such variants have the ability for inducing cytotoxic T cells. An example of a variant peptide is a variant peptide consisting of an amino acid sequence derived from the amino acid sequence as shown in SEQ ID NO: 1, 2, 3, 4, or 5 by deletion, substitution, insertion, or addition of one to several amino acids. The term "one to several" used herein refers to 1 to 5, preferably 1 to 3, and more preferably 1 or 2. Amino acid deletion is carried out by selectively deleting arbitrary amino acid(s) from the amino acid sequence as shown in SEQ ID NO: 1, 2, 3, 4, or 5. Amino acid addition is carried out by adding one to several amino acids to the N terminus or C terminus of the amino acid sequence as shown in SEQ ID NO: 1, 2, 3, 4, or 5. An example of amino acid substitution is the conservative amino acid substitution described above.

A peptide contained in the pharmaceutical composition of the present invention may be in the form of a pharmaceutically acceptable salt. Examples of pharmaceutically acceptable salts include: a salt of an alkali metal, such as lithium, potassium, or sodium; a salt of an alkaline earth metal, such as calcium or magnesium; a salt of an organic base, such as methylamine, ethylamine, or ethanolamine; a salt of an organic acid, such as formic acid, propionic acid, fumaric acid, maleic acid, succinic acid, tartaric acid, citric acid, malic acid, oxalic acid, benzoic acid, methanesulfonic acid, or benzenesulfonic acid; and a salt of an inorganic acid, such as hydrochloric acid, phosphoric acid, acetic acid, sulfuric acid, or hydrobromic acid. Preferable examples include a salt of acetic acid and a salt of hydrochloric acid.

The pharmaceutical composition of the present invention comprises an effective amount of peptides. The term "effective amount" refers to an amount of peptides that is sufficient for the pharmaceutical composition to exert the desirable effects. A person skilled in the art can adequately select an effective amount in accordance with peptide type. In the case of T cell epitope peptides, for example, an effective amount can be determined as the sufficient amount to significantly increase T cells that react specifically to the cells presenting a T cell epitope peptide in the body of a subject to which the pharmaceutical composition had been administered as compared to that in the body of a subject to which no pharmaceutical composition had been administered or that of the subject prior to administration. In the case of T cell epitope peptides, the effective peptide amount is 0.1 mg to 100 mg, preferably 0.25 mg to 50 mg, more preferably 0.5 mg to 20 mg, further preferably 0.5 mg to 10 mg, and most preferably 1 mg to 5 mg. When the pharmaceutical composition of the present invention is prepared in the form of an injection preparation, the peptide concentration in the pharmaceutical composition of the present invention ranges from 0.1 mg/ml to 100 mg/ml, preferably from 0.25 mg/ml to 50 mg/ml, more preferably from 0.5 mg/ml to 20 mg/ml, further preferably from 0.5 mg/ml to 10 mg/ml, and most preferably from 1 mg/ml to 5 mg/ml.

Alternatively, the effective peptide amount may be determined in terms of molar quantity. In the case of T cell epitope peptides, for example, the effective peptide amount ranges from 0.1 µmol to 100 µmol, preferably from 0.25 µmol to 50 µmol, more preferably from 0.5 µmol to 20 µmol, further preferably from 1 µmol to 10 µmol, and most preferably from 1 µmol to 5 µmol. When the pharmaceutical composition of the present invention is prepared in the form of an injection preparation, the peptide concentration in the pharmaceutical composition of the present invention ranges from 0.1 µmol/ml to 100 µmol/ml, preferably from 0.25 µmol/ml to 50 µmol/ml, more preferably from 0.5 µmol/ml to 20 µmol/ml, further preferably from 0.5 µmol/ml to 10 µmol/ml, and most preferably from 1 µmol/ml to 5 µmol/ml.

When the pharmaceutical composition of the present invention contains a plurality of types of peptides, such plurality of types of peptides are each contained in effective amounts in the pharmaceutical composition. In such a case, a person skilled in the art can adequately determine the effective amount in accordance with peptide type. In the case of T cell epitope peptides, the effective amounts thereof and concentration thereof in the pharmaceutical composition are as described above.

The pharmaceutical composition of the present invention comprises a basic amino acid and/or a base, in addition to peptides. Thus, the pharmaceutical composition of the present invention can comprise effective amounts of peptides that are less soluble in water in the form of an aqueous solution. In addition, the pharmaceutical composition can comprise a plurality of types of peptides in effective amounts.

A "basic amino acid" is an amino acid having basic properties. Specific examples thereof include lysine, arginine, ornithine, and histidine. The pharmaceutical composition of the present invention can comprise at least one type of such basic amino acid. A basic amino acid may be an L-, D-, or racemic amino acid. A basic amino acid may be in the form of a salt. Examples of basic amino acids that can be preferably used for the pharmaceutical composition of the present invention include lysine and arginine. According to a preferable embodiment, a basic amino acid is arginine. According to a more preferable embodiment, a basic amino acid is L-arginine. Basic amino acid content in the pharmaceutical composition of the present invention is not particularly limited. In the case of injection preparations, it is 2 mg/ml or more, preferably 5 mg/ml or more, more preferably 7.5 mg/ml or more, further preferably 10 mg/ml or more, and most preferably 15 mg/ml or more. While the upper limit of such content is not particularly limited, the maximal amount described in, for example, *"*Iyakuhin Tenkabutsu Jiten 2007 (Pharmaceutical Additive Dictionary)" (edited by the International Pharmaceutical Excipients Council Japan, Yakuji Nippo Ltd.) can be employed in accordance with the type of basic amino acid to be used and the route of administration. In the case of a hypodermic injection preparation, for example, the maximal amount of L-arginine used per day is 20 mg. When the pharmaceutical composition of the present invention is a hypodermic injection preparation containing L-arginine as a basic amino acid, accordingly, the L-arginine concentration in the pharmaceutical composition of the present invention can be up to 20 mg/ml (when 1 ml of the pharmaceutical composition of the present invention is to be administered).

The pharmaceutical composition of the present invention can comprise a base instead of or in combination with a basic amino acid. The term "base" used herein refers to a substance that produces a hydroxide ion in an aqueous solution, according to the Arrenius definition. A base is a substance that accepts a proton according to the Brønsted-Lowry definition. A base is a substance that donates electron pairs and forms double bonds according to the Lewis definition. Specific examples include sodium hydroxide, sodium carbonate, sodium bicarbonate, triethanolamine, trometamol, and meglumine. Examples of bases that can be preferably used for the pharmaceutical composition of the present invention include sodium carbonate and sodium bicarbonate. Sodium carbonate and sodium bicarbonate may be in the form of hydrates. According to a preferable embodiment, a base is sodium carbonate or a hydrate thereof (e.g., Na₂CO₃·10H₂O). Base content in the pharmaceutical composition of the present invention is not particularly limited. In the case of an injection preparation, it is 0.5 mg/ml or more, preferably 1 mg/ml or more, more preferably 2 mg/ml or more, further preferably 3 mg/ml or more, and most preferably 5 mg/ml or more. While the upper limit of such content is not particularly limited, the maximal amount described in, for example, "Iyakuhin Tenkabutsu Jiten 2007 (Pharmaceutical Additive Dictionary)" (edited by the International Pharmaceutical Excipients Council Japan, Yakuji Nippo Ltd.) can be employed in accordance with the type of base to be used and the route of administration. In the case of a hypodermic injection preparation, for example, the maximal amount of sodium carbonate decahydrate (i.e., Na₂CO₃·10H₂O) used per day is 20.8 mg (i.e., 7.7 mg of sodium carbonate (Na₂CO₃)). When the pharmaceutical composition of the present invention is a hypodermic injection preparation comprising sodium carbonate as a base, accordingly, the sodium carbonate decahydrate concentration can be 20.8 mg in the pharmaceutical composition of the present invention; that is, the sodium carbonate concentration can be up to 7.7 mg/ml (when 1 ml of the pharmaceutical composition of the present invention is to be administered).

According to a preferable embodiment, the pharmaceutical composition of the present invention comprises one or more types of peptides as active ingredients, a basic amino acid, and a base. According to a more preferable embodiment, the pharmaceutical composition of the present invention comprises one or more types of peptides as active ingredients, arginine, and sodium carbonate. According to a further preferable embodiment, the pharmaceutical composition of the present invention comprises one or more types of T cell epitope peptides as active ingredients, arginine, and sodium carbonate.

The pharmaceutical composition of the present invention can comprise additives described in "Iyakuhin Tenkabutsu Jiten 2007 (Pharmaceutical Additive Dictionary)" (Yakuji Nippo Ltd.), according to need. Examples of additives include suspending agents (e.g., benzalkonium chloride, sodium lauryl sulfate, lauryl aminopropionic acid, glyceryl monostearate, polyvinyl alcohol, polyvinyl pyrrolidone, methylcellulose, hydroxymethylcellulose, and hydroxyethylcellulose), dispersants (e.g., sodium citrate, light aluminum oxide, polysorbate, Macrogol, dextrin, low-substituted hydroxypropylcellulose, and hydroxypropylcellulose), emulsifiers (e.g., vaseline, propylene glycol, cetanol, lecithin, lanolin, and sodium lauryl sulfate), surfactants (e.g., cetanol, polyoxyethylene cetyl ether, and Lauromacrogol), isotonizing agents (e.g., glucose, D-sorbitol, D-mannitol, glycerine, and sodium chloride), pH modifiers (e.g., buffers, such as phosphate, acetate, carbonate, and citrate buffers, inorganic acids, such as hydrochloric acid, phosphoric acid, and salts thereof, organic acids, such as acetic acid, citric acid, lactic acid, and salts thereof, and hydroxides, such as sodium hydroxide, potassium hydroxide, and calcium hydroxide), soothing agents (e.g., creatinine and benzyl alcohol), and stabilizers (e.g., taurine, amino acid, p-hydroxybenzoic esters, benzyl alcohol, crystalline cellulose, and Macrogol). Such additives can be used alone or in adequate combination.

The pharmaceutical composition of the present invention can be prepared by dissolving peptides in a pharmaceutically acceptable aqueous solvent comprising a basic amino acid and/or a base dissolved therein in accordance with a method known in the art. Additives as described above may be added to the pharmaceutical composition. The pharmaceutical composition of the present invention can be in the form of, for example, capsules, tablets, granules, powders, syrups, emulsions, suppositories, or injection preparations, in accordance with the route of administration.

According to a preferable embodiment, the pharmaceutical composition of the present invention is administered in the form of an injection preparation. An injection preparation can be prepared using a pharmaceutically acceptable aqueous solvent. Examples of solvents include, but are not limited to, water, physiological saline, and phosphate buffer. According to a preferable embodiment, sterile water prepared for injection (i.e., an injection solvent) can be used as a solvent. An injection preparation can comprise adequate additives, such as buffers, isotonizing agents, stabilizers, preservatives, soothing agents, and pH modifiers, as described above. The pH level of an injection preparation can be adjusted with the use of a pH modifier, according to need. When the pharmaceutical composition of the present invention is in the form of an injection preparation, the pH level is preferably from 6.0 to 11.0 and more preferably from 7.0 to 10.0. While pH modifiers are not particularly limited, use of sodium hydroxide and hydrochloric acid is preferable. The pharmaceutical composition of the present invention prepared in the form of an injection preparation can be administered to a subject via, for example, hypodermic injection, percutaneous injection, intravenous injection, or intramuscular injection.

The pharmaceutical composition of the present invention may be provided in the form of a lyophilized formulation. A lyophilized formulation can be prepared by filling a vial with a composition prepared in the form of an injection preparation as described above and lyophilizing the composition. When performing lyophilization, additives, such as monosaccharides (e.g., glucose, erythrose, xylulose, ribulose, sedoheptulose, ribose, and mannose), disaccharides (e.g., maltose, cellobiose, gentiobiose, melibiose, lactose, turanose, sophorose, trehalose, isotrehalose, saccharose, and isosaccharose), sugar alcohol (e.g., sorbitol, ribitol, and mannitol), or polyvinyl pyrrolidone etc. may be added for the purpose of satisfactory production of a lyophilization cake or peptide stabilization. With the use of the pharmaceutical composition of the present invention, a satisfactory lyophilization cake can be produced and peptide stability can be maintained without the addition of the additives as described above. In general, accordingly, addition of such additives is not necessary.

A lyophilized formulation may be reconstituted with the use of an adequate reconstituting solution, so that the resultant can be administered orally or parenterally. According to a preferable embodiment, a lyophilized formulation is reconstituted and then used in the form of an injection preparation. When a lyophilized formulation is used in the form of an injection preparation after it has been reconstituted, the reconstituting solution, such as sterile water prepared for injection (i.e., an injection solvent) or physiological saline, can be used.

The pharmaceutical composition of the present invention can be administered to mammalians, including humans, such as mice, rats, guinea pigs, rabbits, cats, dogs, sheep, goats, pigs, cows, horses, monkeys, baboons, and chimpanzees. The pharmaceutical composition is preferably administered to humans.

A target disease to be treated with the use of the pharmaceutical composition of the present invention can be adequately selected in accordance with the type of peptide contained as an active ingredient. In the case of the T cell epitope peptide, for example, the pharmaceutical composition can be administered to a subject aimed at induction of an immune response to an antigen from which the T cell epitope peptide is derived. When the pharmaceutical composition of the present invention comprises a T cell epitope peptide derived from a tumor-specific antigen, for example, the pharmaceutical composition of the present invention can be used to induce an immune response to a tumor in the body of the subject. In such a case, the pharmaceutical composition of the present invention can be administered to the subject with the aim of treatment or prevention of cancer.

According to another embodiment of the present invention, the present invention provides the pharmaceutical composition aimed at induction of an immune response to a tumor, comprising one or more types of T cell epitope peptides derived from tumor-specific antigens as active ingredients, a basic amino acid, and/or a base. According to another embodiment, the pharmaceutical composition of the present invention is an agent for inducing an immune response to a tumor that comprises one or more types of T cell epitope peptides derived from tumor-specific antigens as active ingredients, a basic amino acid, and/or a base. According to a further embodiment of the present invention, the present invention provides the pharmaceutical composition aimed at prevention or treatment of cancer, comprising one or more types of T cell epitope peptides derived from tumor-specific antigens as active ingredients, a basic amino acid, and/or a base.

When the pharmaceutical composition of the present invention comprises one or more types of T cell epitope peptides derived from tumor-specific antigens, the pharmaceutical composition of the present invention may be administered in the form of a cancer vaccine. According to another embodiment of the present invention, the pharmaceutical composition is a cancer vaccine comprising one or more types of T cell epitope peptides derived from tumor-specific antigens as active ingredients, a basic amino acid, and/or a base.

A tumor or cancer to which the pharmaceutical composition of the present invention is applied is, without particular limitation, adequately determined in accordance with the type of T cell epitope peptide contained in such pharmaceutical composition. Examples of tumors or cancers to which the pharmaceutical composition of the present invention is applicable include, but are not limited to, bladder cancer, breast cancer, uterine cervical cancer, uterine body cancer, bile duct cancer, colon cancer, esophageal cancer, gastric cancer, hepatic cancer, lung cancer, osteosarcoma, synovial sarcoma, ovarian cancer, pancreatic cancer, prostate cancer, renal cancer, soft tissue tumor, testicular tumor, mesothelial tumor, head and neck cancer, skin cancer, lymphoma, chronic myelocytic leukemia, and acute myelocytic leukemia.

When the pharmaceutical composition of the present invention comprises T cell epitope peptides as active ingredients, the pharmaceutical composition of the present invention may comprise an adjuvant for enhancing an immune response, in addition to the ingredients described above. The term "adjuvant" used herein refers to a substance that enhances an immune response to an immunogenic protein upon administration thereof simultaneously (or in succession) with such protein. Examples of adjuvants that can be used include, but are not limited to, Freund's incomplete adjuvant, Freund's complete adjuvant, GM-CSF and other immunostimulatory cytokines, saponins, saponin derivatives, lipopolysaccharides, lipopeptides, lactoferrin, CpG oligonucleotides, bacterial DNA, and imidazoquinoline. Adjuvants described in a literature (e.g., Clin. Microbiol. Rev., 7: 277-289, 1994) can also be used. Such adjuvants may be used alone or in combinations of two or more.

A hydrophilic adjuvant can be added to the pharmaceutical composition of the present invention before or after peptides have been dissolved in an aqueous solution containing a basic amino acid and/or a base. In contrast, an oil adjuvant is preferably added to the pharmaceutical composition of the present invention after peptides have been dissolved in an aqueous solution. In such a case, it is preferable that an adjuvant be added to the pharmaceutical composition and the resultant be agitated to prepare an emulsion. For example, the pharmaceutical composition of the present invention can be prepared in the form of an emulsified preparation by dissolving peptides in an aqueous solution containing a basic amino acid and/or a base, sterilizing the resulting solution via filtration, and then mixing the resultant with an oil adjuvant, followed by agitation. When the pharmaceutical composition of the present invention is in the form of a lyophilized formulation, alternatively, it can be prepared in the form of an emulsion by reconstituting such lyophilized formulation, and mixing the resultant with an oil adjuvant, followed by agitation. An emulsified preparation or an emulsion with an oil adjuvant can be administered to a subject via, for example, hypodermic injection, percutaneous injection, intravenous injection, or intramuscular injection. According to a preferable embodiment, an emulsified preparation or emulsion is administered to a subject via hypodermic injection. A typical example of an oil adjuvant that can be used for the pharmaceutical composition of the present invention is, but is not limited to, Freund's incomplete adjuvant.

### 2. Method for producing the pharmaceutical composition of the present invention

The present invention provides a method for producing the pharmaceutical composition of the present invention. The pharmaceutical composition of the present invention can be produced by dissolving a basic amino acid and/or a base in a pharmaceutically acceptable aqueous solvent to prepare an aqueous solution comprising a basic amino acid and/or a base, dissolving peptides in the resulting aqueous solution, and formulating the resultant in accordance with a method known in the art. Accordingly, the method for producing the pharmaceutical composition of the present invention comprises a step of dissolving one or more types of peptides in an aqueous solution comprising a basic amino acid and/or a base.

For example, a basic amino acid (e.g., L-arginine) and a base (e.g., sodium carbonate or a hydrate thereof) are dissolved in a pharmaceutically acceptable aqueous solvent, such as sterile water prepared for injection (e.g., Water for Injection defined by the Japanese Pharmacopoeia), so as to prepare an aqueous solution. In such a case, concentrations of a basic amino acid and a base in the aqueous solution can be adequately determined in accordance with, for example, types of a basic amino acid and a base, solubility of peptides in water, and the final dosage form.

When a basic amino acid is L-arginine, for example, its concentration can be from 0 mg/ml to 100 mg/ml. The lower limit of the concentration is preferably 2 mg/ml, 5 mg/ml, 7.5 mg/ml, 10 mg/ml, or 15 mg/ml. The upper limit of the concentration may be determined with reference to the maximal amount described in, for example, "Iyakuhin Tenkabutsu Jiten 2007 (Pharmaceutical Additive Dictionary)" (edited by the International Pharmaceutical Excipients Council Japan, Yakuji Nippo Ltd.), and the concentration in the aqueous solution may be determined in accordance with the deduced final dose. When the pharmaceutical composition contains T cell epitope peptides, for example, the composition is mixed with an equivalent amount of an oil adjuvant, the mixture is agitated to prepare an emulsion, and 1 ml of the emulsion is administered to a subject via hypodermic injection, in general. When a basic amino acid is L-arginine, for example, the maximal amount thereof to be used per day via hypodermic injection is 20 mg, according to "Iyakuhin Tenkabutsu Jiten 2007 (Pharmaceutical Additive Dictionary)." When a basic amino acid is L-arginine, accordingly, the L-arginine concentration in the aqueous solution can be 40 mg/ml or less.

When a base is sodium carbonate or a hydrate thereof, for example, the sodium carbonate concentration can be from 0 mg/ml to 20 mg/ml. The lower limit of the concentration is preferably 0.5 mg/ml, 1 mg/ml, 2 mg/ml, 3 mg/ml, or 5 mg/ml. The upper limit of the concentration may be determined with reference to the maximal amount described in, for example, "Iyakuhin Tenkabutsu Jiten 2007 (Pharmaceutical Additive Dictionary)" (edited by the International Pharmaceutical Excipients Council Japan, Yakuji Nippo Ltd.), and the concentration in the aqueous solution may be determined in accordance with the deduced final dose. When the pharmaceutical composition contains T cell epitope peptides, for example, the composition is mixed with the equivalent amount of an oil adjuvant, the mixture is agitated to prepare an emulsion, and 1 ml of the emulsion is administered to a subject via hypodermic injection, in general. When a base is sodium carbonate decahydrate (Na₂CO₃·10H₂O), the maximal amount thereof to be used per day via hypodermic injection is 20.8 mg, according to "Iyakuhin Tenkabutsu Jiten 2007 (Pharmaceutical Additive Dictionary)" (i.e., 7.7 mg of sodium carbonate (Na₂CO₃)). When a base is sodium carbonate decahydrate, accordingly, concentration thereof in the aqueous solution can be 41.6 mg/ml or less (i.e., 15.4 mg/ml of sodium carbonate).

After an aqueous solution containing a basic amino acid and/or a base is prepared, one or more types of peptides are dissolved in the resulting aqueous solution. When two or more types of peptides are to be dissolved, all types of peptides or some peptides may be simultaneously dissolved. However, it is preferable that peptides be dissolved separately one by one. In general, it is not necessary to consider the order of dissolving peptides. When dissolving peptides, the pH level may be adjusted with the aid of a pH modifier, according to need. A preferable pH range is from 8.0 to 11.0, although the pH level is not limited thereto. In general, the pH level of an aqueous solution is within the above range after peptides have been dissolved, and accordingly, it is not necessary to adjust the pH level.

Concentrations of peptides in the aqueous solution can be adequately determined in accordance with dosage forms, so as to bring the final dose to an effective amount. In the case of T cell epitope peptides, for example, the effective peptide amount is generally from 0.1 mg to 100 mg. In many cases, a pharmaceutical composition comprising T cell epitope peptides is mixed with the equivalent amount of an oil adjuvant, the mixture is agitated to prepare an emulsion, and 1 ml of the emulsion is administered to a subject via hypodermic injection. Thus, peptide concentration in the aqueous solution can be set to an arbitrary level between 0.2 mg/ml and 200 mg/ml. In the case of T cell epitope peptides, typically, peptide concentration in the aqueous solution can be set to an arbitrary level between 1 mg/ml and 20 mg/ml.

Alternatively, the effective peptide amounts may be determined in terms of molar quantity. In the case of T cell epitope peptides, for example, the effective peptide amount is generally 0.1 µmol to 100 µmol. In many cases, a pharmaceutical composition comprising T cell epitope peptides is mixed with the equivalent amount of an oil adjuvant, the mixture is agitated to prepare an emulsion, and 1 ml of the emulsion is administered to a subject via hypodermic injection. Thus, peptide concentration in the aqueous solution can be set to an arbitrary level between 0.2 µmol/ml and 200 µmol/ml. In the case of T cell epitope peptides, typically, peptide concentration in the aqueous solution can be set to an arbitrary level between 1 µmol/ml and 20 µmol/ml.

When two or more types of peptides are to be dissolved, the peptide concentrations in the aqueous solution may be the same or different. When all peptide concentrations are the same, such concentrations may be identical in terms of weight or moles. It is sufficient if the pharmaceutical composition of the present invention comprises all types of peptides in effective amounts. Thus, the ratio of each peptide to be contained is not particularly limited.

After peptides have been dissolved in the aqueous solution, the formulation can be prepared in accordance with a method known in the art. Before or after peptides have been dissolved, the additives and/or adjuvants as described above may be added, according to need.

In the case of an injection preparation, for example, it can be prepared by sterilizing a peptide solution prepared in the manner described above via filtration, filling the resultant in an ampule, a vial, a plastic container, or other adequate container, and hermetically sealing such container. Filter sterilization can be carried out with the use of, for example, a filter for aseptic filtration with a pore diameter of 0.22 µm.

In the case of a lyophilized formulation, for example, it can be prepared by sterilizing a peptide solution prepared in the manner described above via filtration, and filling the resultant in an ampule, a vial, a plastic container, or other adequate container, followed by lyophilization. After the completion of lyophilization, the pressure is recovered, and the container is hermetically sealed. A person skilled in the art can select adequate lyophilization conditions.

When a peptide is a T cell epitope peptide, the pharmaceutical composition of the present invention may be prepared in the form of an emulsion preparation comprising a peptide and an oil adjuvant. For example, an emulsion preparation can be prepared by sterilizing a peptide solution prepared in the manner described above via filtration, and mixing the resultant and the filter-sterilized oil adjuvant and agitating the mixture under aseptic conditions. In general, the mixing ratio of the peptide solution and the oil adjuvant is 1:1, but the mixing ratio is not limited thereto. When an emulsion is produced in a pharmaceutical plant and such, for example, an emulsifier sterilized with steam can be used. Also, an emulsion may be prepared in a hospital or another institution in which a target patient to whom the pharmaceutical composition of the present invention is to be administered is staying. In hospitals or other institutions, emulsion can be prepared by mixing the injection preparation prepared in the manner described above and an oil adjuvant and agitating the mixture with the use of syringes or the like immediately before to 24 hours before the administration. Alternatively, the lyophilized formulation prepared in the manner described above is reconstituted, and an emulsion may be prepared using syringes or the like, as with the case of an injection preparation.

### 3. The kit of the present invention

The present invention also provides a kit comprising (a) a lyophilized formulation of the pharmaceutical composition of the present invention and (b) a reconstituting solution for the lyophilized formulation of the pharmaceutical composition of the present invention.

The pharmaceutical composition of the present invention contained in the kit of the present invention in the form of a lyophilized formulation may be any of the pharmaceutical compositions described above. A pharmaceutical composition contained in the kit of the present invention preferably comprises T cell epitope peptides, and more preferably T cell epitope peptides derived from the tumor-specific antigens. The lyophilized formulation may comprise one or more types of T cell epitope peptides.

The reconstituting solution contained in the kit of the present invention is not particularly limited, and sterile water prepared for injection (an injection solvent), physiological saline, or the like can be used. The reconstituting solution may comprise a peptide different from the peptides contained in the lyophilized formulation dissolved therein.

According to a preferable embodiment, the kit of the present invention comprises a lyophilized formulation comprising one or more peptides each consisting of the amino acid sequence as shown in SEQ ID NO: 1, 2, 3, or 4 and a reconstituting solution. According to another preferable embodiment, the kit of the present invention comprises a lyophilized formulation comprising one or more peptides each consisting of the amino acid sequence as shown in SEQ ID NO: 1, 2, 3, or 4 and a reconstituting solution comprising a peptide consisting of the amino acid sequence as shown in SEQ ID NO: 5. An example of such kit comprises a lyophilized formulation comprising all peptides each consisting of the amino acid sequence as shown in SEQ ID NO: 1, 2, 3, or 4 and a reconstituting solution comprising a peptide consisting of the amino acid sequence as shown in SEQ ID NO: 5.

In addition to the lyophilized formulation (a) and the reconstituting solution (b) described above, the kit of the present invention can comprise (c) one or more types of adjuvants. An adjuvant is not particularly limited, and an oil adjuvant, such as Freund's incomplete adjuvant or Freund's complete adjuvant, can be used.

A lyophilized formulation contained in the kit of the present invention is reconstituted with the aid of a reconstituting solution before it is administered to a subject. It is preferable that the final concentration of each peptide in the reconstituted composition be at least 0.2 mg/ml. According to a preferable embodiment, reconstitution is carried out so as to adjust the final concentration of each peptide to an arbitrary level between 1 mg/ml and 20 mg/ml. When equimolar amounts of peptides are contained in the lyophilized formulation, the lyophilized formulation may be reconstituted so as to adjust the final concentration of each peptide to an arbitrary level between 1 µmol/ml and 20 µmol/ml.

A reconstituted composition can be administered to a subject in the form of, for example, an injection preparation. When the kit of the present invention comprises an adjuvant, the reconstituted composition may be mixed with the adjuvant and the mixture is agitated with the use of, for example, a syringe, and the resultant may then be administered to a subject. In the case of an oil adjuvant, the reconstituted composition may be mixed with the adjuvant and the mixture is agitated with the use of, for example, a syringe, and thereby an emulsion may be prepared. In such a case, the adjuvant is preferably mixed with the equivalent amount of the reconstituted composition, although the amount is not limited thereto. The resulting emulsion can be administered to a subject in the form of an injection preparation. In case of a pharmaceutical composition comprising T cell epitope peptides, for example, 1 ml of the emulsion can be administered to a subject via hypodermic injection or other means.

The present invention also provides a kit comprising (a) the pharmaceutical composition of the present invention and (b) one or more types of adjuvants. In such a case, the pharmaceutical composition of the present invention contained in the kit is preferably an aqueous pharmaceutical composition. The pharmaceutical composition preferably comprises one or more types of T cell epitope peptides, and more preferably T cell epitope peptides derived from tumor-specific antigens. An adjuvant is not particularly limited, and an oil adjuvant, such as Freund's incomplete adjuvant or Freund's complete adjuvant, can be preferably used. The pharmaceutical composition may be mixed with the adjuvant and agitated with the use of, for example, a syringe, and thereby an emulsion can be prepared. The resulting emulsion can be administered to a subject in the form of an injection preparation. In case of a pharmaceutical composition comprising T cell epitope peptides, for example, 1 ml of the emulsion can be administered to a subject via hypodermic injection or other means.

The pharmaceutical composition of the present invention, a lyophilized formulation thereof, a reconstituting solution, and/or an adjuvant are preferably hermetically sealed in an adequate container and contained in the kit of the present invention in such state. Examples of adequate containers include a bottle, a vial, a syringe, and a test tube. A container may have two separate compartments. A container can be formed of a variety of materials, such as a glass or plastic. Preferably, a label is provided on the surface of the container. A label can display descriptions concerning, for example, applicable diseases and administration. For example, a label may show a method for reconstitution of a lyophilized formulation, a method for preparation of an emulsion, a method for administration of an emulsion, and so on.

The kit of the present invention may include components other than those described above. For example, the kit can include other components that are commercially preferable or preferable from the viewpoint of a user, such as a buffer, a diluent, an excipient, a filter, a needle, a syringe, and instructions concerning the method of use.

Hereafter, the present invention is described with reference to the examples and the test examples, although the present invention is not limited thereto.

### Examples

### Peptides for injection preparations

The peptides shown in Table 1 were synthesized for injection preparations. As shown in Table 1, these peptides were deduced to have pI values of 5.2 to 10.4, and their degrees of solubility were deduced to differ from each other. These peptides are derived from tumor-specific antigens that have been confirmed to have the ability for inducing cytotoxic T cells.

### Injection solvent

Pharmaceutical water defined by the Japanese Pharmacopoeia was used as an injection solvent.

**Table 1**

| Peptides | Amino acid sequence | SEQ ID NO: | Predicted pI (average pKa) |
|---|---|---|---|
| Peptide 1 | SYGVLLWEI | 1 | 6.75 (6.48) |
| Peptide 2 | EYYELFVNI | 2 | 7.90 (6.80) |
| Peptide 3 | KVYLRVRPLL | 3 | 10.4 (9.72) |
| Peptide 4 | IYTWIEDHF | 4 | 5.20 (6.05) |
| Peptide 5 | RYCNLEGPPI | 5 | 9.60 (8.08) |
| Peptide 6 | RFVPDGNRI | 6 | 9.09 (8.02) |
| Peptide 7 | SYRNEIAYL | 7 | 9.45 (8.10) |
| Peptide 8 | EYCPGGNLP | 8 | 9.67 (7.38) |
| Peptide 9 | VYGIRLEHF | 9 | 7.60 (7.53) |
| Peptide 10 | DYLNEWGSRF | 10 | 6.95 (7.13) |

### (Comparative Example 1) Preparation of single peptide injection preparations using injection solvent

In order to prepare formulations containing one of the peptides shown in Table 1, each of the peptides shown in Table 1 were added to 1 ml of an injection solvent. The resultants were agitated at room temperature so as to dissolve the entire amounts of the peptides added, and thereby colorless and clear solutions of each peptide were obtained. Thereafter, the peptide solutions were sterilized via filtration (with the use of a 0.22-µm filter) to obtain injection preparations.

The amounts of peptides added varied within the range of 2 mg to 20 mg, and injection preparations were prepared at each concentration. In the case of Peptides 3, 5, 6, 8, and 9, it was possible to prepare the injection preparations regardless of the amounts of peptides added. In contrast, in the case of Peptides 1, 2, 4, 7, and 10, it was not possible to prepare the injection preparations regardless of the amounts of peptides added. For these peptides, peptide concentrations that enable preparation of injection preparations were investigated. As a result, in the case of Peptide 7, it was possible to prepare the injection preparation at a concentration of 0.5 mg/ml. In the case of other peptides, however, it was not possible to prepare the injection preparations even at a concentration of 0.08 mg/ml.

### (Example 1) Preparation of single peptide injection preparations using sodium carbonate solution

41 mg of sodium carbonate decahydrate (Na₂CO₃·10H₂O) was dissolved in 1 ml of an injection solvent to obtain an injection solvent containing sodium carbonate. In order to prepare formulations of the each peptide that was not dissolved in an injection solvent in Comparative Example 1, Peptides 1, 2, 4, 7, and 10 were separately added to 1 ml of injection solvents containing sodium carbonate. The resultants were agitated at room temperature so as to dissolve the entire amounts of the added peptides, and thereby colorless and clear solutions of each peptide were obtained. Thereafter, the peptide solutions were sterilized via filtration (with the use of a 0.22-µm filter) to obtain injection preparations

The amounts of peptides added varied within the range of 5 mg to 20 mg, and injection preparations were prepared at each concentration. In the case of Peptides 2, 4, 7, and 10, it was possible to prepare the injection preparations regardless of the amounts of peptides added. In the case of Peptide 1, it was confirmed that the injection preparation could be obtained with the addition of 9 mg of peptide at the maximum. All the obtained injection preparations had pH levels within adequate range for injection (Table 2).

### (Example 2) Preparation of single peptide injection preparations using arginine solution

40 mg of L-arginine was dissolved in 1 ml of an injection solvent to obtain an injection solvent containing arginine. In order to prepare formulations of each peptide that was not dissolved in an injection solvent in Comparative Example 1, Peptides 1, 2, 4, 7, and 10 were separately added to 1 ml of injection solvents containing arginine. The resultants were agitated at room temperature so as to dissolve the entire amounts of the peptides added, and thereby colorless and clear solutions of each peptide were obtained. Thereafter, the peptide solutions were sterilized via filtration (with the use of a 0.22-µm filter) to obtain injection preparations.

The amounts of peptides added varied within the range of 5 mg to 20 mg, and injection preparations were prepared at each concentration. In the case of Peptides 2, 4, 7, and 10, it was possible to prepare the injection preparations regardless of the amounts of peptides added. In the case of Peptide 1, it was confirmed that the injection preparation could be obtained with the addition of 6 mg of peptide at the maximum. All the obtained injection preparations had pH levels within adequate range for injection (Table 2).

### (Example 3) Preparation of injection preparations using sodium carbonate/arginine solution

41 mg of sodium carbonate decahydrate and 40 mg of L-arginine were dissolved in 1 ml of an injection solvent to obtain an injection solvent containing sodium carbonate and arginine. In order to prepare formulations containing one of the peptides shown in Table 1, the peptides shown in Table 1 were separately added to 1 ml of injection solvents containing sodium carbonate and arginine. The resultants were agitated at room temperature so as to dissolve the entire amounts of the peptides added, and colorless and clear solutions of each peptide were obtained. Thereafter, the peptide solutions were sterilized via filtration (with the use of a 0.22-µm filter) to obtain injection preparations.

The amounts of peptides added varied within the range of 5 mg to 20 mg, and injection preparations were prepared at each concentration. In the case of Peptides 2 and 4 to 10, it was possible to prepare the injection preparations regardless of the amounts of peptides added. In the case of Peptide 3, it was confirmed that the injection preparation could be obtained with the addition of 15 mg of peptide at the maximum. All the obtained injection preparations had pH levels within adequate range for injection (Table 2).

The results of Examples 1 to 3 and Comparative Example 1 are summarized in Table 2. Even when injection preparations of particular peptides could not be prepared with the use of a general injection solvent, injection preparations of such peptides were obtained with the use of an injection solvent containing sodium carbonate, an injection solvent containing arginine, or an injection solvent containing sodium carbonate and arginine. Among these three types of injection solvents, the injection solvent containing sodium carbonate and arginine is considered to be the most preferable solvent for injection because it enables preparation of injection preparations containing any of the tested peptides at a concentration of 10 mg/ml or higher.

**Table 2**

| Peptides | Comparative Example 1 (injection solvent) | Example 1 (Na₂CO₃) | Example 2 (Arg) | Example 3 (Na₂CO₃/Arg) |
|---|---|---|---|---|
| Peptide 1 | × | 9 mg/ml (9.85) | 6 mg/ml (10.17) | 10 mg/ml (10.41) |
| Peptide 2 | × | ⊚ (8.90) | ⊚ (9.03) | ⊚ (9.67) |
| Peptide 3 | ⊚ | - | - | 15 mg/ml |
| Peptide 4 | × | ⊚ (8.88) | ⊚ (8.95) | ⊚ (9.69) |
| Peptide 5 | ⊚ | - | - | ⊚ (10.20) |
| Peptide 6 | ⊚ | - | - | ⊚ (10.55) |
| Peptide 7 | 0.5 mg/ml | ⊚ (9.18) | ⊚ (9.68) | ⊚ (9.97) |
| Peptide 8 | ⊚ | - | - | ⊚ (9.80) |
| Peptide 9 | ⊚ | - | - | ⊚ (9.84) |
| Peptide 10 | × | ⊚ (9.35) | ⊚ (9.87) | ⊚ (10.14) |

| | | | | |
|---|---|---|---|---|
| ⊚: 20 mg/ml or more; ×: 0.08 mg/ml or less; ( ): pH | | | | |

### (Example 4) Preparation of a combination peptide injection preparation using arginine solution

In order to prepare an injection preparation containing combination of the peptides shown in Table 1, the 10 types of peptides shown in Table 1 were added in an amount of 1 mg each to 1 ml of the injection solvent containing arginine as used in Example 2. The entire amount of one peptide was dissolved upon addition thereof to obtain a colorless and clear solution, and another peptide was then added thereto. After the 10 types of peptides had completely dissolved, the resulting peptide solution was sterilized via filtration (with the use of a 0.22 µm filter) to obtain an injection preparation. The resulting injection preparation had a pH level of 9.38, which was within adequate range for injection.

Subsequently, preparation of another injection preparation containing combination of the peptides was attempted in the same manner as described above, except that the amount of each peptide added was changed to 2 mg. When Peptide 3 was added after addition of Peptide 1 and Peptide 2, the solution yielded a white turbidity and a colorless and clear solution could not be obtained. Thus, no other peptides were added thereafter.

### (Example 5) Preparation of a combination peptide injection preparation using sodium carbonate solution

In order to prepare an injection preparation containing combination of the peptides shown in Table 1, the 10 types of peptides shown in Table 1 were added in an amount of 2 mg each to 1 ml of the injection solvent containing sodium carbonate as used in Example 1. The addition of each peptide was conducted in the same manner as in Example 4. Since a colorless and clear solution was obtained upon completion of addition of the 10 types of peptides, and further 2 mg of Peptide 2 was added thereto. As a result, the solution yielded a white turbidity, and a colorless and clear solution could not be obtained.

### (Example 6) Preparation of a combination peptide injection preparation using sodium carbonate/arginine solution

In order to prepare an injection preparation containing combination of the peptides shown in Table 1, the 10 types of peptides shown in Table 1 were added in an amount of 2 mg each to 1 ml of the injection solvent containing sodium carbonate and arginine as used in Example 3. The addition of each peptide was conducted in the same manner as in Example 4. Since a colorless and clear solution was obtained upon completion of addition of the 10 types of peptides, and further 2 mg of Peptide 2 was added thereto, and a colorless and clear solution was obtained. The resulting solution was sterilized via filtration (with the use of a 0.22-µm filter) to obtain an injection preparation. The resulting injection preparation had a pH level of 9.72, which was within adequate range for injection.

The results of Examples 4 to 6 are summarized in Table 3. With the use of the injection solvent containing arginine, it was possible to prepare a combination preparation containing all 10 types of peptides if the amount of each peptide added was 1 mg. With the use of the injection solvent containing sodium carbonate, it was possible to prepare a combination preparation containing all 10 types of peptides even if the amount of each peptide added was 2 mg. With the use of the injection solvent containing sodium carbonate and arginine, it was possible to prepare a combination preparation even if the peptide was further added after all peptides had been added in an amount of 2 mg. Accordingly, even in the case of combination peptide preparations, the injection solvent containing sodium carbonate and arginine is considered to be the most preferable injection solvent for injection.

**Table 3**

| Peptides | Example 4-1 (Arg) (1 mg each) | Example 4-2 (Arg) (2 mg each) | Example 5 (Na₂CO₃) (2 mg each) | Example 6 (Na₂CO₃/Arg) (2 mg each) |
|---|---|---|---|---|
| Peptide 1 | ○ | ○ | ○ | ○ |
| Peptide 2 | ○ | ○ | ○ | ○ |
| Peptide 3 | ○ | × | ○ | ○ |
| Peptide 4 | ○ | - | ○ | ○ |
| Peptide 5 | ○ | - | ○ | ○ |
| Peptide 6 | ○ | - | ○ | ○ |
| Peptide 7 | ○ | - | ○ | ○ |
| Peptide 8 | ○ | - | ○ | ○ |
| Peptide 9 | ○ | - | ○ | ○ |
| Peptide 10 | ○ | - | ○ | ○ |
| Peptide 2 | - | - | × | ○ |

| | | | | |
|---|---|---|---|---|
| ○: Dissolved; ×: White Turbid | | | | |

### (Example 7) Preparation of lyophilized formulation of combination peptide preparation

Preparation of a lyophilized formulation was attempted with the use of Peptide 1, Peptide 2, Peptide 3, and Peptide 4.

107.7 g of L-arginine and 110.4 g of sodium carbonate hydrate were dissolved in 5,500 ml of an injection solvent. Peptide 1, Peptide 2, Peptide 3, and Peptide 4 were dissolved therein in amounts of 6.2 g, 7.5 g, 8.7 g, and 8.0 g, respectively. Thereafter, the total amount of the solution was adjusted to 7,000 ml with the addition of an injection solvent, and a combination peptide solution was obtained. The combination peptide solution was sterilized via filtration with the use of a 0.22 µm PVDF filter, a vial that had been washed and sterilized was filled with 2.6 ml of the resultant, and the vial was half-stoppered with a rubber cap that had been washed and sterilized (2 µmol of each peptide/2.6 ml). Following lyophilization, the vial was fully stoppered and the aluminum cap seaming was conducted. Thus, a lyophilized formulation was produced.

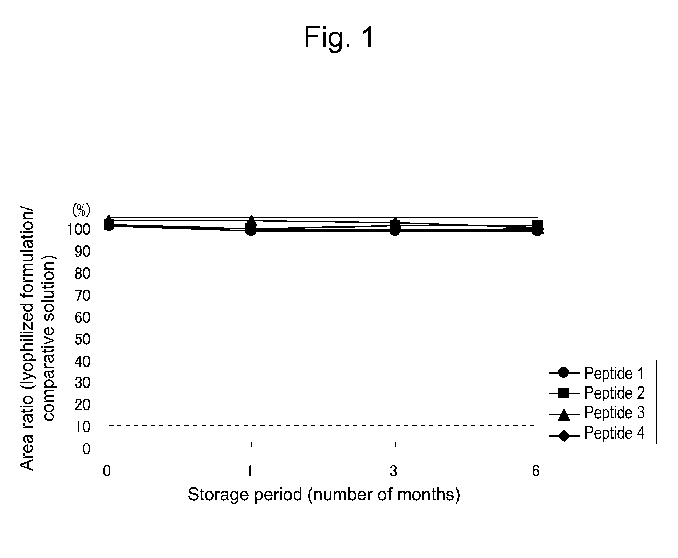

The lyophilized formulation was stored at 25 degrees C and at a humidity of 60%, and the peptide contents in the vial were measured with the elapse of time to ascertain the stability of the lyophilized formulation. Measurement of peptide contents in the vial was carried out by resolubilizing the lyophilized formulation with the aid of a physiological saline and detecting the each peptide in the resulting solution by HPLC. The results of measurement are shown in Table 4 and Fig. 1. Peptide content in the vial was expressed as a value relative to a peak area designated to be 100%, which was detected in a comparative solution prepared to have the same concentration as that of the lyophilized formulation at the time of the preparation.

**Table 4**

| | 0 months | 1 month | 3 months | 6 months |
|---|---|---|---|---|
| Peptide 1 | 101.0% | 98.7% | 98.9% | 98.6% |
| Peptide 2 | 101.8% | 99.5% | 101.3% | 101.4% |
| Peptide 3 | 103.7% | 103.4% | 102.7% | 100.1% |
| Peptide 4 | 101.2% | 99.6% | 99.4% | 99.5% |

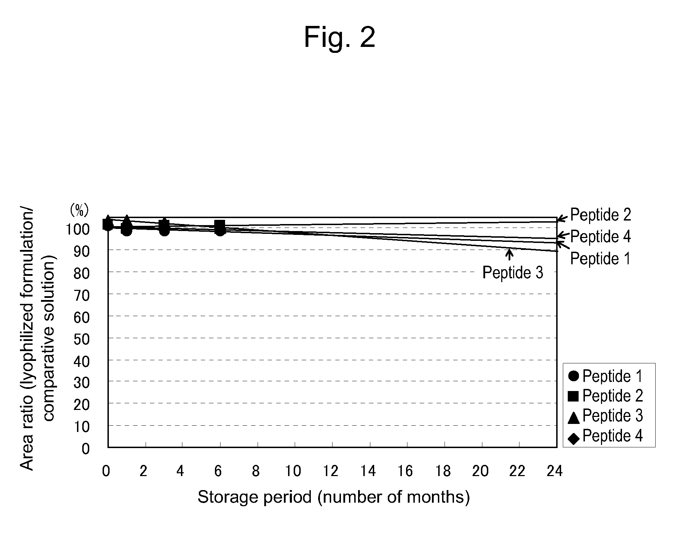

The results shown in Table 4 and Fig. 1 demonstrate that peptides are not substantially degraded and remain stable in the lyophilized formulation 6 months later. Further, the results shown above were subjected to regression analysis, so as to predict stability 24 months later. As a result, it was predicted that the peptides would remain stable in the lyophilized formulation 24 months later (Fig. 2). Thus, the lyophilized formulation prepared by the method of the present invention was found to be sufficiently stable as a pharmaceutical product.

### (Example 8) Resolubilization and emulsification of lyophilized formulation

The lyophilized formulation prepared in Example 7 is supposed to be used as a peptide vaccine preparation for cancer therapy. A peptide vaccine for cancer therapy is often mixed with an oil adjuvant and administered to a patient in the form of an emulsion, so as to enhance induction of anti-cancer immunity. Thus, the lyophilized formulation prepared in Example 7 was subjected to a resolubilization test and an emulsification test.

First, 2.6 ml of physiological saline was added to a vial of the lyophilized formulation prepared in Example 7 in an attempt to achieve resolubilization. The results showed that the lyophilized formulation had resolubilized immediately. Next, on the assumption that the lyophilized formulation would be mixed with an equivalent amount of an oil adjuvant, 1.3 ml of physiological saline was added to the vial in an attempt to achieve resolubilization. The results showed that the lyophilized formulation had resolubilized immediately with the addition of 1.3 ml of physiological saline.

In order to investigate whether a lyophilized formulation could be resolubilized with physiological saline containing a peptide dissolved therein, further, 1.3 ml of physiological saline containing Peptide 5, which was prepared as a dimer, was added at 2 µmol/ml to the vial in an attempt to achieve resolubilization of the lyophilized formulation. The results showed that the lyophilized formulation had resolubilized immediately with the addition of 1.3 ml of physiological saline containing Peptide 5 dissolved therein. If a plurality of types of peptides are to be administered to a patient, accordingly, whether peptides are to be contained in a lyophilized formulation or dissolved in a resolubilizing solution may be determined in accordance with the properties of the peptides.

Subsequently, the resolubilized solution that had been prepared by resolubilization of the lyophilized formulation with the addition of 1.3 ml of physiological saline containing Peptide 5 dissolved therein was mixed with 1.3 ml of Montanide® ISA 720VG (SEPPIC), and the resultant was agitated with the use of two syringes connected to each other in an attempt to achieve emulsification. The resulting emulsion was evaluated by a drop test and found to be a homogenous emulsion. The evaluation results demonstrate that the lyophilized formulation prepared in Example 7 can be resolubilized, and administered to a patient in the form of an emulsion with the aid of an oil adjuvant.

### Industrial Applicability

The present invention is applicable in the field of production of pharmaceutical compositions comprising peptides and, in particular, peptide vaccine preparations for cancer therapy comprising a plurality of types of T cell epitope peptides.

All publications, patents, and patent applications cited herein are incorporated herein by reference in their entirety.

## Claims

1. A pharmaceutical composition comprising one or more types of peptides as active ingredients and a basic amino acid and/or a base.

2. The pharmaceutical composition according to [1], wherein the basic amino acid are one basic amino acid or a combination of two or more basic amino acids selected from the group consisting of arginine, lysine, ornithine, histidine, hydroxylysine, and a salt of any thereof.

3. The pharmaceutical composition according to claim 1 or 2, wherein the base are one base or a combination of two or more bases selected from the group consisting of sodium carbonate, potassium carbonate, sodium bicarbonate, potassium bicarbonate, sodium hydroxide, potassium hydroxide, triethanolamine, trometamol, and meglumine.

4. The pharmaceutical composition according to any one of claims 1 to 3, wherein the peptide is a T cell epitope peptide.

5. The pharmaceutical composition according to any one of claims 1 to 3, wherein the peptide is a peptide composed of two or more types of T cell epitope peptides linked to each other directly or via a linker.

6. The pharmaceutical composition according to claim 4 or 5, wherein the T cell epitope peptide is one or more types of peptides each consisting of the amino acid sequence as shown in SEQ ID NO: 1, 2, 3, 4, or 5.

7. The pharmaceutical composition according to claim 4 or 5, wherein the T cell epitope peptide is all types of peptides each consisting of the amino acid sequence as shown in SEQ ID NO: 1, 2, 3, or 4.

8. The pharmaceutical composition according to any one of claims 4 to 7, which is used for induceing an immune response to a tumor.

9. The pharmaceutical composition according to claim 8, which is used for prevention or treatment of cancer.

10. The pharmaceutical composition according to any one of claims 1 to 9, which is prepared in the form of a parenteral formulation.

11. The pharmaceutical composition according to claim 10, wherein the parenteral formulation is an injection preparation.

12. A lyophilized formulation of the pharmaceutical composition according to any one of claims 1 to 11.

13. A method for producing a pharmaceutical composition, which is the injection preparation according to claim 11, comprising a step of dissolving one or more types of peptides in an aqueous solution comprising a basic amino acid and/or a base.

14. A method for producing the lyophilized formulation according to claim 12, which comprises the following steps of:
(1) dissolving one or more types of peptides in an aqueous solution comprising a basic amino acid and/or a base; and
(2) lyophilizing the peptide solution prepared in (1).

15. The method for producing the lyophilized formulation according to claim 14, wherein the peptide is all types of peptides each consisting of the amino acid sequence as shown in SEQ ID NOs: 1, 2, 3, or 4.

16. A kit comprising (a) and (b) below:
(a) the lyophilized formulation according to claim 12; and
(b) a reconstituting solution for the lyophilized formulation of (a).

17. A kit comprising (a) and (b) below:
(a) a lyophilized formulation of the pharmaceutical composition according to claim 6 or 7; and
(b) a reconstituting solution for the lyophilized formulation of (a) comprising a peptide consisting of the amino acid sequence as shown in SEQ ID NO: 5.

18. The kit according to claim 16 or 17, which further comprises (c) below:
(c) one or more types of adjuvants.

19. A kit comprising (a) and (b) below:
(a) the pharmaceutical composition according to any one of claims 1 to 11; and
(b) one or more types of adjuvants.


SEQUENCE LISTING

<110>  ONCOTHERAPY SCIENCE, Inc.

<120>  Parmaceutical composition comprising peptides

<130>  PH-5524-PCT

<150>  JP 2012-072352
<151>  2012-03-09


<160>  10

<170>  PatentIn version 3.5

<210>  1
<211>  9
<212>  PRT
<213>  Artificial Sequence

<220>
<223>  T cell epitope

<400>  1

Ser Tyr Gly Val Leu Leu Trp Glu Ile
1               5


<210>  2
<211>  9
<212>  PRT
<213>  Artificial Sequence

<220>
<223>  T cell epitope

<400>  2

Glu Tyr Tyr Glu Leu Phe Val Asn Ile
1               5


<210>  3
<211>  10
<212>  PRT
<213>  Artificial Sequence

<220>
<223>  T cell epitope

<400>  3

Lys Val Tyr Leu Arg Val Arg Pro Leu Leu
1               5                   10


<210>  4
<211>  9
<212>  PRT
<213>  Artificial Sequence

<220>
<223>  T cell epitope

<400>  4

Ile Tyr Thr Trp Ile Glu Asp His Phe
1               5


<210>  5
<211>  10
<212>  PRT
<213>  Artificial Sequence

<220>
<223>  T cell epitope

<400>  5

Arg Tyr Cys Asn Leu Glu Gly Pro Pro Ile
1               5                   10


<210>  6
<211>  9
<212>  PRT
<213>  Artificial Sequence

<220>
<223>  T cell epitope

<400>  6

Arg Phe Val Pro Asp Gly Asn Arg Ile
1               5


<210>  7
<211>  9
<212>  PRT
<213>  Artificial Sequence

<220>
<223>  T cell epitope

<400>  7

Ser Tyr Arg Asn Glu Ile Ala Tyr Leu
1               5


<210>  8
<211>  9
<212>  PRT
<213>  Artificial Sequence

<220>
<223>  T cell epitope

<400>  8

Glu Tyr Cys Pro Gly Gly Asn Leu Phe
1               5

<210>  9
<211>  9
<212>  PRT
<213>  Artificial Sequence

<220>
<223>  T cell epitope

<400>  9

Val Tyr Gly Ile Arg Leu Glu His Phe
1               5


<210>  10
<211>  10
<212>  PRT
<213>  Artificial Sequence

<220>
<223>  T cell epitope

<400>  10

Asp Tyr Leu Asn Glu Trp Gly Ser Arg Phe
1               5                   10